# EUROPEAN PATENT SPECIFICATION

(11) **EP 3 999 147 B1**
(45) Date of publication and mention of the grant of the patent: **22.04.2026**
(21) Application number: 20843328.4
(22) Date of filing: 22.06.2020
(51) Int. Cl.: A61M 5/142, A61M 5/145, A61M 5/168

(54) **UNILATERAL-DRIVEN MEDICAL DEVICE WITH INFUSION AND DETECTION INTEGRATED**
UNILATERAL GESTEUERTES IMPLANTIERBARES MEDIZINPRODUKT MIT INTEGRIERTER INFUSION UND DETEKTION
DISPOSITIF MÉDICAL À ENTRAÎNEMENT UNILATÉRAL AVEC PERFUSION ET DÉTECTION INTÉGRÉES

(30) Priority: 19.07.2019 WO PCT/CN2019/096673; 31.12.2019 WO PCT/CN2019/130445; 31.12.2019 WO PCT/CN2019/130442
(43) Date of publication of application: 25.05.2022
(73) Proprietor: Medtrum Technologies Inc., Shanghai 201203 (CN)
(72) Inventor: YANG, Cuijun, Shanghai 201203 (CN)
(74) Representative: Becker, Eberhard
(86) International application number: PCT/CN2020/097317
(87) International publication number: WO 2021/012852

(56) References cited:
- WO-A2-2011/064780
- WO-A2-2011/064780
- CN-A- 101 563 022
- CN-A- 103 260 678
- CN-A- 104 168 935
- CN-B- 103 463 695
- US-A1- 2003 199 824
- US-A1- 2003 199 824
- US-A1- 2003 236 498
- US-A1- 2007 073 228
- US-A1- 2019 117 881
- US-A1- 2019 117 881

## Description

### TECHNICAL FIELD

The present invention mainly relates to the field of medical instruments, in particular to a unilateral-driven medical device with infusion and detection integrated.

### BACKGROUND

Diabetes is mainly a metabolic disease caused by abnormal human pancreatic function. Diabetes is a lifelong disease. At present, medical technology cannot cure diabetes. It can only control the occurrence and development of diabetes and its complications by stabilizing blood glucose. The normal human pancreas automatically monitors changes in the body's blood glucose levels and automatically secretes the required insulin. At present, the medical device for stabilizing blood glucose works by dynamically monitoring the blood glucose changes of the human body by a glucose sensor implanted in the subcutaneous tissue of the human body; and continuously accurately infusing insulin into the subcutaneous tissue of the human body through a medical cannula implanted in the subcutaneous tissue of the human body.

This method requires separately inserting glucose sensor and infusion cannula under the human skin. Even though there are some devices that can integrate the sensor probe and the infusion cannula into one device, the sensor and cannula still need to be separately inserted at different positions, increasing the risk of infection.

Therefore, there is a need in the prior art for a unilateral-driven medical device with infusion and detection integrated that can perform both detection and infusion at the same time.

WO 2011/064780 A2 discloses an analyte monitoring and fluid dispensing system. CN 103 463 695 B discloses a quantitative supply mechanism for trace medicine liquid. US 2003/0199824 A1 discloses a dispenser for patient infusion device. US 2007/0073228 A1 discloses a dispensing fluid from an infusion pump system.

### BRIEF SUMMARY OF THE INVENTION

The invention is provided by appended set of claims. The following disclosure serves a better understanding of the present invention.

Compared with the prior arts, the technical solution of the present invention has the following advantages:
In the unilateral-driven medical device with infusion and detection integrated disclosed herein, an infusion cannula provided with at least two detecting electrodes. The infusion cannula performs analyte detection and drug infusion at the same time. Once the puncture is performed at one position, the analyte detection and the drug infusion can be completed simultaneously, reducing the risk of the user's infection. Secondly, when the infusion cannula is installed to the working position, the infusion cannula connects with the drug infusion unit to allow the drugs to flow through the infusion cannula into the body, and the different electrodes are electrically connected to different electrically connective regions inputting the analyte data signal to the program unit. With this design method, after the user attaches the medical device to the skin surface, the mounting unit for installing the infusion cannula is pressed. When the infusion cannula is installed to the working position, the medical device can begin to work. This approach reduces the user's pre-using steps and improves the user experience.

Furthermore, when the electrode located on the outer wall surface of the inner layer cannula is covered in whole or in part by the outer layer cannulas, the material of the outer layer cannulas wall is permeable membrane or a semi-permeable membrane. The cannula wall material is selected from a permeable membrane or a semi-permeable membrane to ensure the required analyte passes through the cannula wall to the electrode surface. It can improve the flexibility of electrode position design without affecting the detection.

Furthermore, a plurality of electrodes constitute one or more electrode combinations, each electrode combination includes working electrode and auxiliary electrode, and the program unit selects one or more electrode combinations to detect the body fluid analyte data. On the one hand, when a combination of electrodes fails to detect, the program unit can select other electrode combinations for detection according to the situation to ensure the detection process of the body fluid signal is uninterrupted. On the other hand, the program unit can select multiple electrode combinations to work at the same time, performing statistical analysis on multiple sets of data of the same parameter at the same time, improving the detection accuracy of the analyte data, and then issue a more accurate infusion signal.

Furthermore, the drug infusion unit comprises a plurality of infusion subunits, the plurality of infusion subunits being electrically connected to the output end respectively, and the program unit controlling whether each infusion subunit delivers drugs. Different drugs are reserved in different infusion subunits, and the program unit sends different drug infusion instructions to different infusion subunits to achieve precise control of the analyte level in body fluid.

Furthermore, the power unit and the reset unit are linear actuators. The force of the linear actuator can be controlled by current. When the current is constant, the force exerted by the linear actuator is constant. Therefore, the linear actuator can exert stable and controllable force, thus making the drug infusion proceed smoothly.

Furthermore, the reset unit at least includes a spring, an elastic sheet, an elastic plate, an elastic rod, or an elastic reset rubber. When the reset unit is an elastic member, the reset unit can work without consuming additional energy, which reduces the power consumption of the medical device and saves the production cost.

### BRIEF DESCRIPTION OF THE DRAWINGS

FIG.1 is a flow chart of the operation of a unilateral-driven medical device with infusion and detection integrated according to an embodiment of the present invention;
FIG.2 is a schematic view of the main structures of the drug infusion unit according to an embodiment of the present invention;
FIG.3a - FIG.3c are top views of the driving portion pushing the wheel teeth according to different embodiments of the present invention;
FIG.4a - FIG.4c are schematic views of the power unit, the reset unit and the driving unit cooperating with each other according to different embodiments of the present invention;
FIG.5a - FIG.5b are schematic views of that the pulling direction of the power unit is not parallel to the advancing direction of the screw according to another embodiment of the present invention, and FIG.5b is the top view of FIG.5a;
FIG.6 is a schematic view of a driving unit including two driving units according to an embodiment of the present invention;
FIG.7 is a schematic view of a driving unit including two driving units according to another embodiment of the present invention;
FIG.8a - FIG.8b are schematic views of two driving portions of one driving unit cooperating with two sub-wheels respectively according to yet another embodiment of the present invention;
FIG.9a - FIG.9b are schematic views of two driving portions of one driving unit cooperate with the same driving wheel according to yet another embodiment of the present invention, and FIG.9b is a schematic view of the driving unit in FIG.9a;
FIG.10a is a schematic view of an infusion cannula of a unilateral-driven medical device with infusion and detection integrated in a pre-installation position according to one embodiment of the present invention;
FIG.10b is a schematic view of an infusion cannula of a unilateral-driven medical device with infusion and detection integrated in a working position according to an embodiment of the present invention;
FIG.11a - FIG.11b are top views of a unilateral-driven medical device with infusion and detection integrated according to different embodiments of the present invention;
FIG.12a - FIG.12b are partial longitudinal views of an infusion cannula including two electrodes according to one embodiment of the present invention;
FIG.13a - FIG.13c are partial longitudinal views of an infusion cannula and the two electrodes according to another embodiment of the present invention;
FIG.14 is a partial longitudinal view of an infusion cannula provided with three electrodes according to still another embodiment of the present invention;
FIG.15 is a partial longitudinal view of an infusion cannula including an inner layer cannula and one outer layer cannula according to still another embodiment of the present invention.

### DETAILED DESCRIPTION

As described above, in the prior art device, the detection and the infusion are performed separately to control the analyte level in the body fluid, and it is necessary to puncture at multiple positions on the skin, thereby increasing the pain of the user and increasing the risk of infection.

The study found that the cause of the above problems is that the sensor detection device and the drug medical device are two independent units. Or even if the two are designed into a single structure, multiple puncture positions are still required on the body surface.

In order to solve this problem, the present invention provides a unilateral-driven medical device with infusion and detection integrated, the infusion cannula is used for detecting analyte data and a drug infusion channel. And it can perform detection and infusion with only one puncture.

Various exemplary embodiments of the present invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, width, length or distance of certain units may be exaggerated relative to other structures.

The following description of the exemplary embodiments is merely illustrative, and is not intended to be in any way limiting the invention and its application or use. The techniques, methods and devices that are known to those of ordinary skill in the art may not be discussed in detail, but such techniques, methods and devices should be considered as part of the specification.

It should be noted that similar reference numerals and letters indicate similar items in the following figures. Therefore, once an item is defined or illustrated in a drawing, it will not be discussed further in the following description of the drawings.

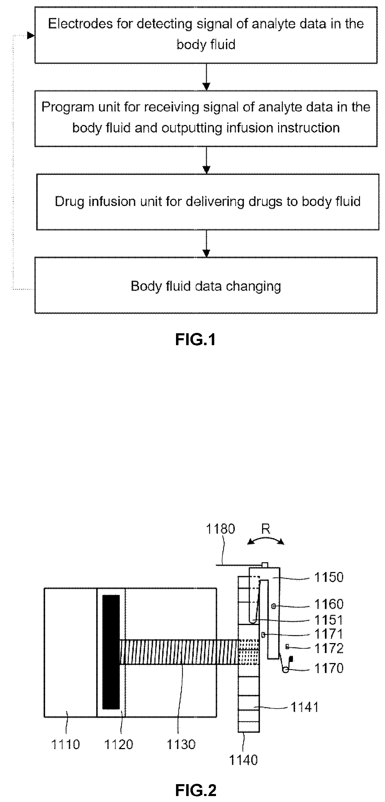

FIG.1 is a flow chart showing the operation of a unilateral-driven medical device with infusion and detection integrated according to an embodiment of the present invention.

The unilateral-driven medical device with infusion and detection integrated of the embodiment of the invention comprises three basic parts: electrodes, a program unit and a drug infusion unit. The body fluid analyte data is obtained by the electrodes and converted into an electrical signal. Electrical signals are passed to the program unit via electrodes and/or electrode leads. After analyzing the body fluid analyte data signal, the program unit, through the power unit or the reset unit, sends a signal to the drug infusion unit controlling whether to perform a drug infusion, thereby stabilizing the body fluid parameters. The body fluid analyte data are detected by the electrodes in real time, and the cycle of detection and infusion is without interruption. This process does not require human intervention and is done directly through program analysis to control the stability of body fluid parameters.

FIG.2 is a schematic view of main structures in the infusion unit according to an embodiment of the present invention.

The internal structure of the infusion unit mainly includes at least one drug storage unit 1110, a piston 1120, a screw 1130, a driving wheel 1140, at least one driving unit 1150, a pivot shaft 1160, a reset unit 1170 and a power unit 1180. In the embodiment of this present invention, the driving unit 1150 is connected to the reset unit 1170 and the power unit 1180, respectively. It should be noted that the connection herein includes mechanical connection or electrical connection.

The drug storage unit 1110 is used for storing liquid drug. Drugs include, but are not limited to, insulin, glucagon, antibiotics, nutrient solutions, analgesics, morphine, anticoagulants, gene therapy drugs, cardiovascular drugs or chemotherapy drugs, etc.

The piston 1120 is used to infuse liquid drug into the body.

The screw 1130 is connected to the piston 1120 and the driving wheel 1140, respectively. In the embodiment of the present invention, the driving wheel 1140 advances the screw 1130 forward by screwing, the screw 1130 then forces the piston 1120, arranged in the drug storage unit 1110, to move forward, so as to achieve the purpose of drug infusion.

The peripheral surface of the driving wheel 1140 is provided with wheel teeth 1141. The wheel teeth 1141 are gear teeth or ratchet teeth. Specifically, in the embodiment of the present invention, for improving driving efficiency, the wheel teeth 1141 are ratchet teeth which can be pushed more easily.

At least one driving portion 1151 is provided on the driving unit 1150 to push the wheel teeth 1141, thereby rotating the driving wheel 1140. The driving unit 1150 is movably connected to the pivot shaft 1160.

The power unit 1180 and the reset unit 1170 cooperate with each other to make the driving unit 1150 rotate reciprocally around the pivot shaft 1160, as shown in the R direction in FIG.2, thereby making the driving portion 1151 move in the advancing direction and reset direction. When the driving unit 1150 performs one reciprocating rotation, the driving wheel 1140 drives the screw 1130 forward one step, and the screw 1130 engages the piston 1120 to infuse one unit of drug.

It should be noted here that the advancing direction of the driving portion 1151 refers to the direction in which the wheel teeth 1141 moves, while the reset direction of the driving portion 1151 is opposite to the advancing direction. During the reset movement, the driving portion 1151 only slides on the surface of the wheel teeth 1141 without pushing them.

In some embodiments of the present invention, the reset unit 1170 at least includes a spring, an elastic piece, an elastic plate, an elastic rod, rubber and other elastic members. It should be noted that the spring herein includes a compression spring, an extension spring, or a torsion spring, etc. Specifically, in the embodiment of the present invention, the reset unit 1170 is a torsion spring which is more conducive to the reset of the driving unit 1150. When the reset unit 1170 is an elastic member, it can work without consuming additional energy, which reduces the power consumption of the medical device and saves the production cost.

In other embodiments of the present invention, the reset unit 1170 includes an electrically driven linear actuator or an electrically heated linear actuator, such as a shape memory alloy. After being energized, the physical form of the material of the linear actuator, like shape memory alloy, changes, which makes shrinkage deformation of the shape memory alloy occur, thereby outputting the driving force. The higher the current is, the larger the shrinkage deformation of the shape memory alloy occurs, and the greater the driving force outputs. Obviously, when the current is constant, the shrinkage deformation of the linear actuator is also constant, so the driving force is. Therefore, the shape memory alloy can output stable and controllable driving force for drug infusion.

The type, material selection or the position of the reset unit 1170 are not specifically limited herein, as long as it can satisfy the condition of making the driving unit 1150 rotate in the reset direction.

The power unit 1180 is an electrically driven linear actuator or an electrically heated linear actuator. By alternately turning on and off, the power unit 1180 outputs force in pulses. Specifically, in the embodiment of the present invention, the power unit 1180 is a shape memory alloy.

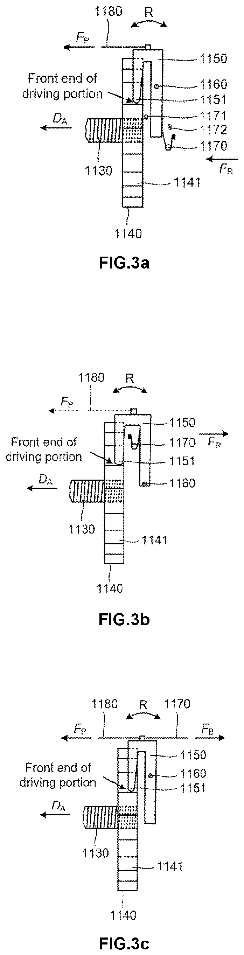

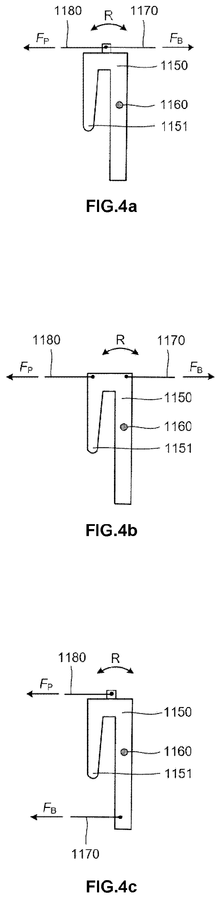

FIG.3a - FIG.3c are top views of the driving portion 1151 pushing the wheel teeth 1141 in different embodiments of the present invention. FIG.4a - FIG.4c are schematic views of the power unit 1180, the reset unit 1170 and the driving unit 1150 cooperating with each other in different embodiments of the present invention.

As shown in FIG.3a and FIG.3b, the principle of the reciprocating rotation of the driving unit 1150 in the embodiment of the present invention is as follows. When the power unit 1180 pulls the driving unit 1150 by force *F*_{P}, the driving unit 1150 rotates counter-clockwise (advancing direction) around the pivot shaft 1160, driving the driving portion 1151 to push the wheel teeth 1141 forward, and thereby making the driving wheel 1140 rotate. The driving wheel 1140 then moves the screw 1130 forward in *D*_{A} direction. The reset unit 1170, as an elastic member, builds a gradually increasing elastic force *F*_{R}. When the power unit 1180 stops applying force and under the action of only the elastic force *F*_{R}, the driving unit 1150 will rotate clockwise (reset direction) around the pivot shaft 1160. At this time, the driving portion 1151 just slides on the surface of the wheel teeth 1141 instead of pushing them, therefore the driving wheel 1140 stops rotating. The driving unit 1150 completes one reciprocating rotation.

As shown in FIG.3b, in another embodiment of the present invention, the reset unit 1170 and the power unit 1180 are disposed on the same side of the pivot shaft 1160. And according to the general technical principles, those skilled in the art can arbitrarily adjust the positional relationship and the connection relationship of the reset unit 1170, the driving unit 1150, and the power unit 1180, which is not specifically limited herein, as long as the above-mentioned rotation processes can be achieved.

As shown in FIG.3c, in yet another embodiment of the present invention, the reset unit 1170 includes an electrically driven linear actuator or an electrically heated linear actuator, such as a shape memory alloy. Although the technical principle that the driving portion 1151 pushes the wheel teeth 1141 is consistent with the foregoing, the driving unit 1150 cannot automatically reset after the driving portion 1151 stops advancing, therefore, the reset unit 1170 is required to provide the reset force *F*_{B} whose direction is opposite with that of *F*_{P}, thereby making the driving unit 1150 rotate reciprocally with the cooperation of the reset unit 1170 and the power unit 1180.

Preferably, as shown in FIG.3a to FIG.3c, in the embodiment of the present invention, the directions of *F*_{P}, *F*_{R} (or *F*_{B}) and *D*_{A} are parallel to one another. This parallel design can make full use of space and optimize the structural relationships inside the infusion unit, making internal structure more compact.

Obviously, those skilled in the art can arbitrarily adjust the directions of the *F*_{P} and *F*_{B} as needed, as long as the conditions for reciprocating rotation of the driving unit 1150 are satisfied, as shown in FIG.4a - FIG.4c.

In other embodiments of the present invention, the direction of *F*_{P} and the direction of *F*_{R} (or *F*_{B}) or the direction of *D*_{A} may not be parallel, which is not specifically limited herein, as long as the purpose of the reciprocating rotation of the driving unit 1150 can be achieved.

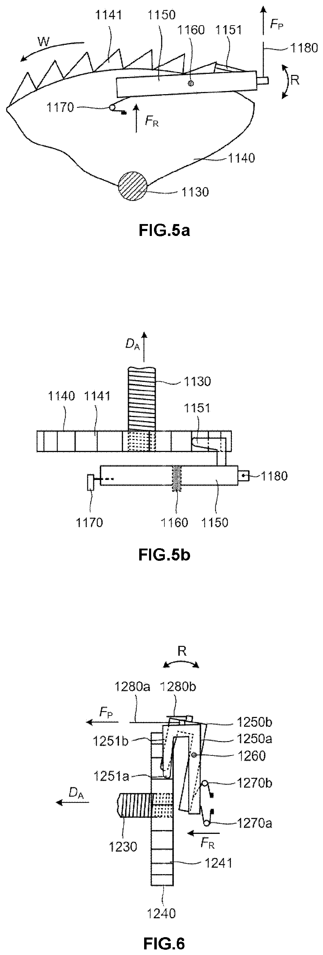

FIG.5a - FIG.5b are schematic views of that the pulling direction of the power unit 1180 is not parallel to the advancing direction of the screw 1130. FIG.5b is the top view of FIG.5a.

The *F*_{P} direction of the power unit 1180 is perpendicular to the forward direction *D*_{A} of the screw 1130. The pivot shaft 1160 and the reset unit 1170 are disposed on the base. As described above, the driving unit 1150, rotating reciprocally in the R direction, drives the driving portion 1151 to push the wheel teeth 1141, making the driving wheel 1140 rotate in the W direction and driving the screw 1130 to advance in the *D*_{A} direction. The driving principle of the driving unit 1150 is consistent with the foregoing embodiment.

In the embodiment of the present invention, blocking walls 1171 and 1172 (as shown in FIG.2 and FIG.3a) that can stop the driving unit 1150 from rotating are also provided in the infusion unit. And an electrical signal may be triggered when the driving unit 1150 contacts the blocking wall 1171 or 1172, allowing the program unit to control the driving force output of the power unit 1180. In another embodiment of the present invention, only the blocking wall 1171 or only the blocking wall 1172 may be provided, so that the rotating terminal in either direction of the driving unit 1150 is controlled by the program unit. The position of the blocking wall 1171 or 1172 is not specifically limited herein, as long as the condition that the driving unit 1150 stops rotating can be satisfied.

In another embodiment of the present invention, no blocking wall is provided (as shown in FIG.3b to FIG.5b), and the rotating terminal of the driving unit 1150 is completely controlled by the program unit.

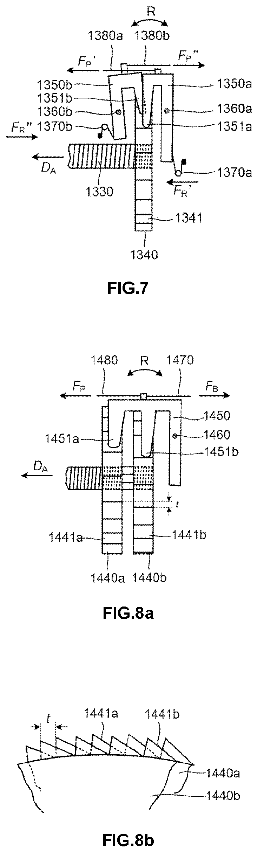

FIG.6 and FIG.7 are schematic views of a driving unit including two driving units according to different embodiments of the present invention.

As shown in FIG.6, the driving unit 1250a rotates reciprocally in the R direction around the pivot shaft 1260 under the action of the linear actuator 1280a and the reset unit 1270a. Similarly, the driving unit 1250b rotates reciprocally in the R direction around the pivot shaft 1260 under the action of the linear actuator 1280b and the reset unit 1270b. In the embodiment of the present invention, the reciprocating rotations of the two driving units do not interfere with each other. Therefore, both the driving unit 1250a and the driving unit 1250b can independently implement the driving method or principle described above.

Preferably, in the embodiment of the present invention, the driving unit 1250a and the driving unit 1250b rotate asynchronously. That is, when the driving portion 1251a of the driving unit 1250a pushes the wheel teeth 1241 to move, the driving portion 1251b of the driving unit 1250b slides on the surface of the wheel teeth 1241. When the driving portion 1251b slides to one position, the program unit controls the linear actuator 1280a to stop outputting driving power to the driving unit 1250a, and in turn controls the linear actuator 1280b to output power to the driving unit 1250b. At this time, the driving unit 1250a rotates in the clockwise direction under the action of the reset unit 1270a, and the driving portion 1251a slides on the surface of the wheel teeth, while the driving portion 1251b pushes the wheel teeth 1241. The driving units 1250a and 1250b are alternately powered to push the same driving wheels 1240.

In the embodiment of the present invention, the pulling force *F*_{P} of the linear actuators 1280a and 1280b, the elastic force *F*_{R} of the reset units 1270a and 1270b and the forward direction *D*_{A} of the screw 1230 are shown in the figures. Like foregoing statement, the direction of the pulling force *F*_{P} is parallel to the forward direction *D*_{A} of the screw 1230.

In the embodiment of the present invention, the types of the reset units 1270a and 1270b can be referred to the above, which will not be repeated herein.

As shown in FIG.7, the driving portions 1351a and 1351b alternately push the wheel teeth 1341, respectively, and the force output by the linear actuators 1380a and 1380b are both controlled by the program unit.

It should be noted that, in the embodiment of the present invention, the direction of the pulling force *F*_{P}' of the linear actuator 1380a and that of the pulling force *F*_{P}" of the linear actuator 1380b are opposite. Obviously, the direction of the resetting force *F*_{R}' of the reset unit 1370a and that of the resetting force *F*_{R}" of the reset unit 1370b are also opposite.

Also, in the embodiment of the present invention, the driving units 1350a and 1350b rotate asynchronously. That is, when the driving portion 1351a of the driving unit 1350a pushes the wheel teeth 1341 forward, the driving portion 1351b of the driving unit 1350b slides on the surface of the wheel teeth 1341. When the driving portion 1351b slides to one position, the program unit controls the linear actuator 1380a to stop outputting power to the driving unit 1350a, and in turn controls the linear actuator 1380b to output power to the driving unit 1350b. The driving unit 1350a resets to the clockwise rotation by the reset unit 1370a, while the driving portion 1351a slides on the surface of the wheel teeth 1341, and the driving portion 1351b pushes the wheel teeth 1341. The driving units 1350a and 1350b alternately pushes the driving wheels 1340.

Similarly, both the driving unit 1350a and the driving unit 1350b can independently implement the driving method or principle described above. And the types of the reset units 1370a and 1370b can be referred to the above, which will not be repeated herein.

It should be noted that, in other embodiments of the present invention, more driving units can be arranged in the driving unit, or more driving portions are disposed on each driving unit, or the driving wheel includes more sub-wheels. Therefore, different driving units respectively push the corresponding sub-wheel to rotate.

FIG.8a and FIG.8b are schematic views of two driving portions 1451a and 1451b of a driving unit 1450 cooperating with two sub-wheels 1440a and 1440b, respectively according to yet another embodiment of the present invention. FIG.8b is a right view of the partial teeth structure of the sub-wheels 1440a and 1440b in FIG.8a.

As shown in FIG.8a and FIG.8b, in the embodiment of the present invention, the driving unit 1450 includes two driving portions 1451a and 1451b disposed left and right, while the driving wheel includes two fixedly connected sub-wheels 1440a and 1440b disposed on the left and right (that is, two sub-wheels can move simultaneously). The driving portions 1451a and 1451b cooperate with the sub-wheels 1440a and 1440b, respectively, and the pivot shaft 1460 is disposed on the same side of two sub-wheels 1440a and 1440b. Both the linear actuator 1480 and the reset unit 1470 of the embodiment of the present invention are shape memory alloys, and the driving portion 1451a or 1451b can respectively push the wheel teeth 1441a or 1441b forward. Their working principles and operating modes are consistent with the foregoing embodiments.

In addition to driving portion 1451a or 1451b operating independently, the embodiment of the present invention can also adjust the distance between the front ends of the driving portions 1451a and 1451b, or adjust the offset degree of the wheel teeth 1441a and 1441b to make two driving portions 1451a and 1451b cooperate with each other. Preferably, in the embodiment of the present invention, the wheel teeth 1441a and 1441b are offset with degree *t*, as shown in FIG.8a and FIG.8b.

Obviously, in the embodiment of the present invention, two driving portions 1451a and 1451b reciprocate synchronously. As shown in FIG.8a, when the previous forward movement is completed, the driving unit 1450 starts a reset rotation, the driving portion 1451a reaches the driving position before the driving portion 1451b, so the driving portion 1451a can be used to start the next forward movement instead. Or the driving unit 1450 continues the reset movement until the driving portion 1451b reaches the next driving position to start the next forward movement.

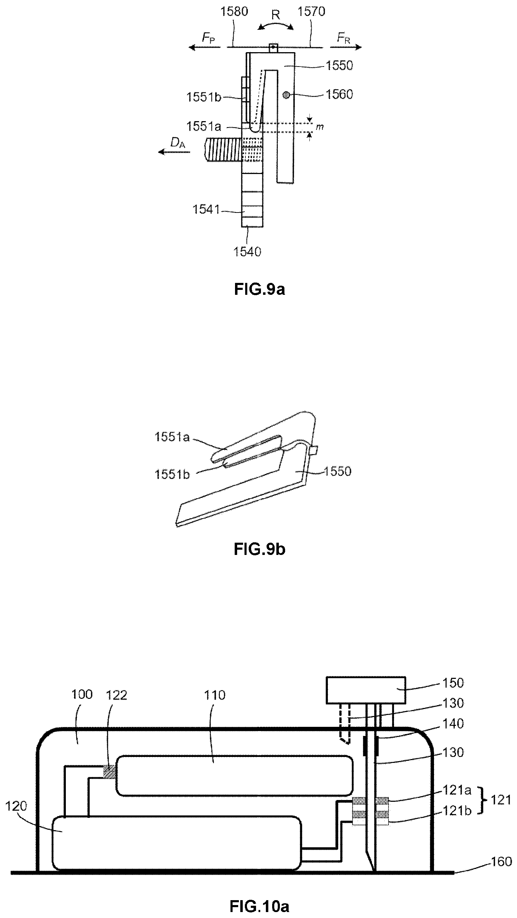

FIG.9a and FIG.9b are still another embodiment of the present invention in which the driving unit 1550 includes two driving portions 1551a and 1551b disposed up and down, and driving portions 1551a and 1551b cooperate with the same driving wheel 1540. FIG.9b is a schematic view of the driving unit 1550 in FIG.9a.

As shown in FIG.9a and FIG.9b, the driving unit 1550 includes two driving portions 1551a and 1551b disposed up and down cooperating with the same driving wheel 1540, so the driving portions 1551a and 1551b reciprocate synchronously. The front ends of the driving portions 1551a and 1551b are not level with a certain distance *m*, therefore, the two cannot simultaneously push the wheel teeth 1541 forward, as shown in FIG.9a. When the driving portion 1551b finishes the last forward movement, the driving unit 1550 performs a reset movement, obviously making the driving portion 1551a reach the next driving position before the driving portion 1551b. The driving portion 1551a can be used to push the wheel teeth 1541 forward to start the next forward movement.

In other embodiments of the present invention, the driving unit may further include more driving portions, such as 3, 4 or more, which is not specifically limited herein.

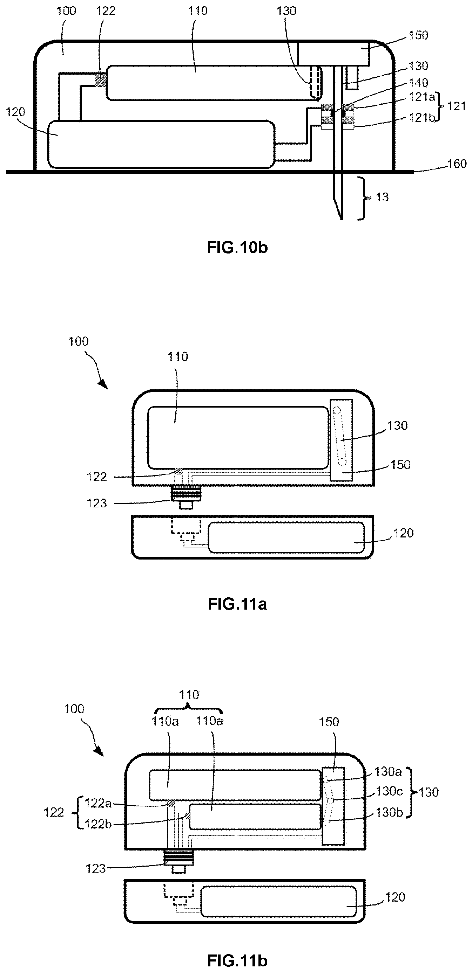

FIG.10a - FIG.10b are views of a medical device 100 according to an embodiment of the present invention, and the medical device 100 is an integral structure. FIG.10a shows the infusion cannula 130 in the pre-installation position while FIG.10b shows the infusion cannula 130 in the working position.

Program unit 120 includes an input end 121 and an output end 122. The input end 121 is used for receiving a body fluid analyte data signal. In the embodiment of the invention, the input end 121 includes electrically connective regions 121a and 121b. When in operation, the electrically connective region is electrically connected to the electrode or electrode lead to receive the analyte signal. In other embodiments of the invention, the input end 121 may also include more electrically connective regions depending on the number of electrodes. The output end 122 is electrically coupled to the power unit or the reset unit, allowing the program unit 120 to effectively control the drug infusion unit 110.

During the use of the unilateral-driven medical device with infusion and detection integrated of the embodiment of the present invention, the infusion cannula 130 can slid relative to the input end 121, while the input end 121 is provided as an elastic member. The elastic member is to ensure an interference fit between the infusion cannula 130 and the input end 121 to avoid poor electrical contact. The elastic member includes: conductive rubber strip, oriented conductive silica gel, conductive ring, conductive ball, etc. When the number of electrodes is relatively large, the electrically connective regions are relatively dense. In this case, according to different structural designs, the elastic members may be one or more combinations of the above.

In an embodiment of the invention, the infusion cannula 130 is mounted on the mounting unit 150. When the infusion cannula 130 is in the pre-installation position, the mounting unit 150 protrudes from the outer surface of the medical device 100, as shown in FIG.10a. When the infusion cannula 130 is installed to the working position, the mounting unit 150 is pressed into the medical device 100 with the top portion integral with the medical device 100 housing, as shown in FIG.10b. Prior to use by users, the mounting unit 150 holds the infusion cannula 130 in the pre-installation position. After the medical device 100 is attached on the surface of the human body, the mounting unit 150 is pressed to insert the infusion cannula under the skin, and the unilateral-driven medical device with infusion and detection integrated can start operation. Compared with other infusion cannula installation methods, the installation method of the embodiment of the invention reduces the steps required for installation, makes the installation more convenient and flexible and improves the user experience.

The manner of setting the infusion cannula 130 in the mounting unit 150 can be various, and is not specifically limited herein. Specifically, in the embodiment of the present invention, the other side of the mounting unit 150 also protrudes from the partial infusion cannula 130 (shown by a dotted line in FIG.10a and FIG.10b) for subsequent connection with the outlet of the drug infusion unit 110 to achieve drug circulation.

In other embodiments of the invention, the infusion cannula 130 further includes an electrical contact region 140 coupled to the input end 121. As shown in FIG.10a, the electrical contact region 140 is not electrically coupled to the input end 121 when the infusion cannula 130 is in the pre-installation position. And the other end of the infusion cannula 130 is also not connected with the drug infusion unit 110 outlet. As shown in FIG.10b, when the infusion cannula 130 is mounted to the working position, one end of the infusion cannula 130 is inserted subcutaneously (indicated by the solid line portion of the infusion cannula in FIG.10b) and the other end (illustrated by the dotted portion of the infusion cannula in FIG.10b) is connected with the outlet of the drug infusion unit 110, thereby establishing a flow path for the drug from the drug infusion unit 110 to the body tissue fluid. At the same time, the electrical contact region 140 reaches the electrically connective region of the input end 121, enabling electrical connection between the program unit 120 and the electrical contact region 140.

It should be noted that even if the infusion cannula 130 and the drug infusion unit 110 are connected, and the input end 121 and the electrical contact region 140 of the infusion cannula 130 are electrically connected, as long as the infusion cannula 130 does not penetrate the skin, the program unit 120 will not enter working mode, so that the unilateral-driven medical device with infusion and detection integrated does not generate any analyte data signal, nor does it issue an instruction to inject drug. Therefore, in other embodiments of the present invention, when the infusion cannula 130 is in the pre-installation position, the electrical contact region 140 may also be electrically connected to the electrically connective region of the input end 121 or the infusion cannula 130 may be coupled to the outlet of the drug infusion unit 110. And there are no specific restrictions herein.

In an embodiment of the invention, a medical tape 160 for attaching the medical device 100 to the skin surface is used to paste the program unit 120, the drug infusion unit 110, the electrode and the infusion cannula 130 as a whole on the skin. When the infusion cannula 130 is installed to the working position, the portion of the infusion cannula 130 that is inserted into the skin is 13.

FIG.11a is a top view of a medical device 100 in accordance with another embodiment of the present invention.

In one embodiment of the invention, the medical device 100 comprises two parts. The program unit 120 is disposed in one part, the drug infusion unit 110 is disposed in another part, and the two parts are electrically connected by the waterproof electrical plug 123. The part of the drug infusion unit 110 can be discarded after being used once, and the part of the program unit 120 can be reused, saving the user's cost.

In other embodiments of the present invention, the medical device 100 may also be composed of more parts, and parts that do not require electrical connection may be connected using a common waterproof plug.

FIG.11b is a top view of a medical device 100 in accordance with another embodiment of the present invention.

In an embodiment of the invention, the medical device 100 comprises two parts, and the drug infusion unit 110 comprises two infusion subunits 110a and 110b. The drug storage units of the infusion subunits 110a and 110b can be used to reserve different drugs, respectively. Infusion subunits 110a and 110b are electrically coupled to outputs 122a and 122b, respectively, allowing the program unit 120 to effectively control the drug infusion unit 110. The outlets of infusion subunits 110a and 110b can be connected with the 130a portion and 130b portion of infusion cannula respectively. 130a and 130b are connected with the 130c portion of infusion cannula, respectively. The 130c portion of the infusion cannula is used to penetrate the skin, thereby establishing a path for the two drugs to flow from the drug infusion unit 110 into the body fluid. That is, the medical device still penetrates the skin only in one position. In the embodiment of the present invention, after the body fluid analyte data signal is transmitted to the program unit 120, program unit 120 can output different infusion signals to different infusion subunits to control whether infusion of drug is required. This method realizes accurate detection and control of body fluid analyte level to stabilize the physiological state of the user.

In other embodiments of the present invention, there may be more infusion subunits according to actual needs, and multiple infusion subunits may be disposed in different parts of the medical device 100. There are no specific restrictions herein.

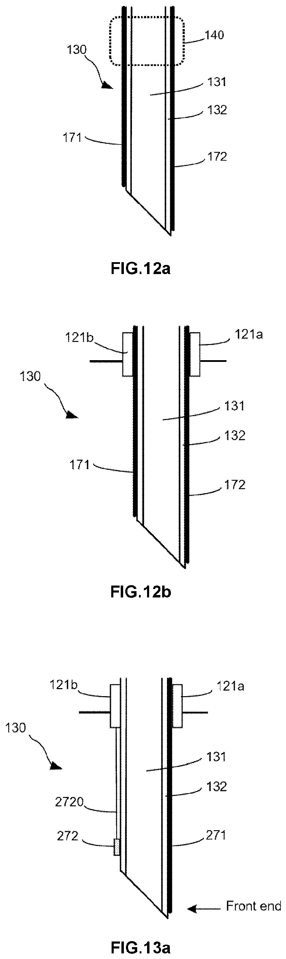

FIG.12a - FIG.12b are partial longitudinal views of the infusion cannula 130 including two electrodes.

In the embodiment of the invention, the medical device 100 includes at least two detecting electrodes that are disposed on the wall of the infusion cannula 130, as shown in FIG.12a. The different electrodes are electrically connected to the electrically connective regions at the position of the dotted frame 140. The cavity 131 of the infusion cannula 130 is used for drug infusion.

In the embodiment of the present invention, the electrodes, such as electrode 171 and electrode 172, are plated on the outer surface of the cannula wall of the infusion cannula 130. The electrode 171 and the electrode 172, electrically insulated from each other, are directly electrically connected to the electrically connective regions 121a and 121b of the input end, respectively, which allows electrical signals of the body fluid analyte data to be transmitted to program unit 120, as shown in FIG.12b. Once the puncture is performed at one position, the analyte detection and the drug infusion can be completed simultaneously, reducing the risk of the user's infection.

It should be noted that, in the embodiment of the present invention, when the infusion cannula 130 is mounted to the working position, a part of the electrode 171 or the electrode 172 is located in the subcutaneous tissue fluid, while another part is located above the skin, so that electrical signals can be transmitted on the electrode. The corresponding electrode arrangements in the other embodiments below have the same function and will not be described in detail later.

In the embodiment of the present invention, the medical device 100 has only two electrodes, the electrode 171 is a working electrode while the electrode 172 is an auxiliary electrode. In another embodiment of the invention, the electrode 171 is an auxiliary electrode while the electrode 172 is a working electrode. The auxiliary electrode is a counter electrode.

In other embodiments of the present invention, more electrodes, which are electrically insulated from each other, may be provided on the surface of the infusion cannula 130.

FIG.13a - FIG.13c are partial longitudinal views of an infusion cannula 130 in accordance with another embodiment of the present invention.

It should be noted that the electrodes or electrode leads in all embodiments of the present invention are coated or plated on the infusion cannula 130, but for ease of marking and description, the electrode leads or electrodes and the infusion cannula will be depicted separately in the FIG.s. The following related structural views are the same as those here, which will not be described in detail below.

In this embodiment, the cannula wall 132 of the infusion cannula 130 provides with the electrode 271 and the electrode 272. And the electrode 271 is directly electrically connected to the electrically connective regions 121a, such as the electrode 171 in FIG.12a. The electrode 272 is disposed at the front end of the infusion cannula 130. And an electrode lead 2720 is used to electrically connect to the electrode 272 and the electrically connective regions 121b. When the infusion cannula 130 is mounted to the working position, the electrode 272 is located on the subcutaneous part of the outer surface of the cannula wall 132, while a part of the electrode 272 is located in the subcutaneous tissue fluid and another part is located above the skin. At this time, the electrode 272 is indirectly electrically connected to the electrically connective regions 121b, sending parameter information to the program unit.

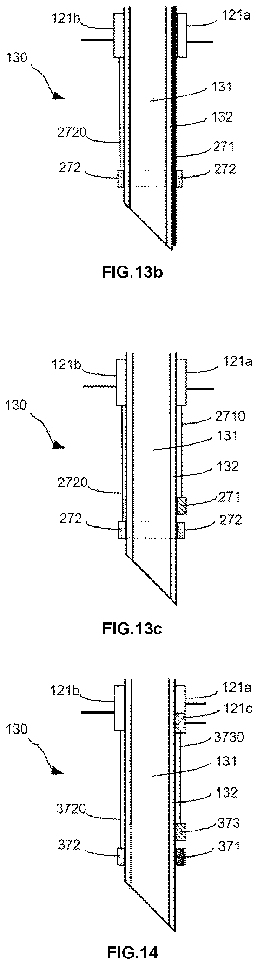

The embodiment of the present invention does not specifically limit the shape of the electrode 272. If the electrode 272 may be ring-shaped, the electrode 272 surrounds the front end of the infusion cannula 130, as shown in FIG.13b. At this time, an insulation layer is provided between the electrode 272 and the electrode 271. As shown in FIG.13c, in yet another embodiment of the present invention, the electrode 271 and the electrode 272 are both provided at the front end of the infusion cannula 130, that is, on the subcutaneous part of the outer surface of the cannula wall. The outer surface of the cannula wall 132 is also provided with an electrode lead 2710 and an electrode lead 2720 that are electrically connected to the electrode 271 and the electrode 272, respectively. When the infusion cannula 130 is installed to the working position, the electrically connective regions 121a and 121b at the input end are electrically connected to the electrode lead 2710 and the electrode lead 2720, respectively. Therefore, the electrode 271 and the electrode 272 are indirectly electrically connected to the input end, transmitting the body fluid parameter signal to the program unit. During detection, both the electrode 271 and the electrode 272 are located in the subcutaneous tissue fluid.

As shown in FIG.13c, the electrode 272 is arranged in a ring shape surrounding a part of the outer surface of the cannula wall 132. The electrode 271 and the electrode 272 may have other shapes, which is not specifically limited herein.

FIG.14 is a partial longitudinal view of an infusion cannula 130 provided with three electrodes in accordance with yet another embodiment of the present invention.

In the embodiment of the present invention, three electrodes are disposed on the infusion cannula 130: the electrode 371, 372 and 373 which are all disposed on the outer surface of the cannula wall 132. Similarly, the surface of the cannula wall 132 is also provided with electrode leads 3720 and 3730 which are electrically connected to the electrode 372 and the electrode 373, respectively. Similarly, the outer surface of the cannula wall 132 is also provided with an electrode lead electrically connected to the electrode 371, but it is not shown in order to simplify the marking. When the infusion cannula 130 is installed to the working position, the electrode lead of the electrode 371, electrode lead 3720 and electrode lead 3730 are electrically connected to the electrically connective regions 121a, 121b, and 121c of the input end, respectively, connecting the input end to each electrode. The shapes of the three electrodes can be various, and there is no specific limitation herein.

In the embodiment of the present invention, in order to simplify the design of the electrically connective region, the elastic member at the input end is an oriented conductive silica gel or a conductive ring. By doping different elements in the silica gel, it is possible to achieve directional conduction, such as horizontal conduction or vertical conductivity. Thus, even if 121a and 121c are adjacent to each other, the two can still be electrically insulated from each other. The electrically connective region 121b may be a conductive rubber strip or a conductive ball or the like, and is not specifically limited herein.

In the embodiment of the present invention, the electrode 371 is a working electrode, and the electrode 372 and the electrode 373 are both auxiliary electrodes. At this time, the electrode 371 and the electrode 372 or the electrode 373 may constitute a different electrode combination, that is, the two electrode combinations share the electrode 371. The program unit 120 can select different electrode combinations to detect body fluid analyte data. After the electrode combination is formed, on the one hand, when a working electrode combination fails to detect, the program unit 120 can select other electrode combinations for detection according to the situation to ensure that the detection process of the body fluid signal is uninterrupted. On the other hand, the program unit 120 can select a plurality of electrode combinations to work simultaneously, perform statistical analysis on multiple sets of data of the same parameter at the same time, improve the accuracy of the analyte data, and thereby output a more accurate drug infusion signal.

In another embodiment of the present invention, the electrode 371, electrode 372, and electrode 373 include an auxiliary electrode and two working electrodes, which can also be arbitrarily selected according to actual needs, which are not specifically limited herein.

As an embodiment of the present invention, the electrode 371 is a working electrode, the electrodes 372 and 373 are auxiliary electrodes which are used as a counter electrode and a reference electrode, respectively, thereby forming a three-electrode system. Similarly, the three electrodes can be arbitrarily selected according to actual needs, which are not specifically limited herein.

Also, in other embodiments of the invention, more electrodes may be provided. The system includes a plurality of working electrodes and a plurality of auxiliary electrodes. At this time, each electrode combination includes at least a working electrode and an auxiliary electrode, and thus a plurality of electrodes may constitute a plurality of electrode combinations. The program unit 120 may select one or more electrode combinations to detect body fluid analyte data, as desired.

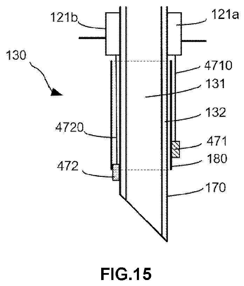

FIG.15 is a partial longitudinal view of an infusion cannula 130 including an inner layer cannula 170 and one outer layer cannula 180 in accordance with yet another embodiment of the present invention.

The cavity 131 of the inner layer cannula 170 is used as a drug infusion channel. The cannula wall of the infusion cannula 130 includes the inner layer cannula wall and the outer layer cannula wall. The electrode 472 is disposed outside the cannula wall of the inner layer cannula 170, while the electrode 471 is provided on the outer surface of the wall of the outer layer cannula 180. At this time, the electrode 472 is disposed in the wall of the infusion cannula 130, that is, the electrode 472 is embedded between the outer layer cannula 180 and the inner layer cannula 170.

In the embodiment of the present invention, the electrode 472 may be partially covered by the outer layer cannula 180 (as shown in FIG.15), or completely covered by the outer layer cannula 180. The electrode 472 is electrically connected to the electrically connective region 121b through an electrode lead 4720, while the electrode 471 is electrically connected to the electrically connective region 121a through an electrode wire 4710. When the electrode 472 is partially or completely covered by the outer layer cannula 180, the wall material of the outer layer cannula 180 is a permeable membrane or a semi-permeable membrane. Such selection can facilitate the body fluid analyte to pass through the wall of the outer layer cannula 180 and to be detected by the electrode, thereby improving the flexibility of electrode position design without affecting the detection.

In another embodiment of the present invention, the electrode 471 and the electrode 472 are both disposed in the wall of the infusion cannula 130, that is, the electrode 471 and the electrode 472, which are completely covered by the outer layer cannula 180, are both embedded between the inner layer cannula 170 and the outer layer cannula 180. At this time, the material of the outer layer cannula 180 is as described above, which makes analytes detected by the electrode through the outer layer cannula 180.

It should be noted that, in other embodiments of the present invention, more layers of outer layer cannulas may be arranged outside the inner layer cannula 170. And as described above, more electrodes can be provided on the infusion cannula 130. According to actual needs, different electrodes can be arranged between different outer layer cannulas. And at least one electrode is disposed between the wall of the inner layer cannula and the outermost cannula.

In addition to embedding electrodes into the wall of the infusion cannula 130, some embodiments of the present invention can also reduce the length of the outer layer cannula 180 in FIG.15, directly exposing the electrode 472 disposed on the outer surface of the inner layer cannula 170 in tissue fluid. In summary, the present invention discloses a unilateral-driven medical device with infusion and detection integrated that has both infusion and detection functions to reduce the number of punctures on the skin. With only one puncture at one position, analyte detection and drug infusion can be completed, reducing the risk of infection.

While the invention has been described in detail with reference to the specific embodiments of the present invention, it should be understood that it will be appreciated by those skilled in the art that the above embodiments may be modified without departing from the scope of the invention. The scope of the invention is defined by the appended claims.

## Claims

1. A unilateral-driven medical device (100) with infusion and detection integrated, **characterized in that**, comprising:
a drug infusion unit (110), including:
at least one drug storage unit (1110);
a screw (1130) connected to a piston (1120) and a driving wheel (1140, 1240, 1340, 1540) provided with a wheel teeth (1141, 1241, 1341, 1441a, 1441b, 1541), respectively, the driving wheel (1140, 1240, 1340, 1540) drives the screw (1130) to move by rotation, pushing the piston (1120), provided in the at least one drug storage unit (1110), forward;
at least one driving unit (1150, 1250a, 1250b, 1350a, 1350b, 1450, 1550) cooperating with the driving wheel (1140, 1240, 1340, 1540), the at least one driving unit (1150, 1250a, 1250b, 1350a, 1350b, 1450, 1550) includes at least one driving portion (1151, 1251a, 1251b, 1351a, 1351b, 1451a, 1451b, 1551a, 1551b);
a power unit (1180) and a reset unit (1170) connected to the at least one driving unit (1150, 1250a, 1250b, 1350a, 1350b, 1450, 1550), wherein:
when the power unit (1180) exerts a force on the at least one driving unit (1150, 1250a, 1250b, 1350a, 1350b, 1450, 1550), the at least one driving unit (1150, 1250a, 1250b, 1350a, 1350b, 1450, 1550) rotates around a pivot shaft (1160), driving the at least one driving portion (1151, 1251a, 1251b, 1351a, 1351b, 1451a, 1451b, 1551a, 1551b) to push the wheel teeth (1141, 1241, 1341, 1441a, 1441b, 1541), thus rotating the driving wheel (1140, 1240, 1340, 1540);
when the reset unit (1170) exerts a force on the at least one driving unit (1150, 1250a, 1250b, 1350a, 1350b, 1450, 1550) alone, the at least one driving unit (1150, 1250a, 1250b, 1350a, 1350b, 1450, 1550) performs a reset rotation without the at least one driving portion (1151, 1251a, 1251b, 1351a, 1351b, 1451a, 1451b, 1551a, 1551b) pushing the wheel teeth (1141, 1241, 1341, 1441a, 1441b, 1541), thus making the driving wheel (1140, 1240, 1340, 1540) stop rotating;
a program unit (120) comprising an input end (121) and an output end (122), and the input end (121) comprises a plurality of electrically connective regions (121a, 121b, 121c) for receiving signals of an analyte data in a body fluid, after the output end (122) is electrically connected to the power unit (1180) or the reset unit (1170), according to received signals of the analyte data in the body fluid, the program unit (120), through the power unit (1180) or the reset unit (1170), controls the rotation of the at least one driving unit (1150, 1250a, 1250b, 1350a, 1350b, 1450, 1550) to implement drug infusion; and
an infusion cannula (130) provided with at least two electrodes (171, 172, 271, 272, 371, 372, 373, 471, 472), the infusion cannula (130) is a drug infusion channel, the at least two electrodes (171, 172, 271, 272, 371, 372, 373, 471, 472) are disposed on/in a cannula wall (132), when the infusion cannula (130) is installed to a working position, the infusion cannula (130) is connected with the drug infusion unit (110), a drug is then capable of being injected into a body through the infusion cannula (130), and the different electrodes (171, 172, 271, 272, 371, 372, 373, 471, 472) are electrically connected to the different electrically connective regions (121a, 121b, 121c) respectively, inputting signal of the analyte data in the body fluid to the program unit (120),
wherein the electrode (472) located on the outer wall surface of the inner layer cannula (170) is covered in whole or in part by the at least one outer layer cannula (180), material of the at least one outer layer cannula (180) walls is a permeable membrane or a semi-permeable membrane.

2. The unilateral-driven medical device (100) with infusion and detection integrated of claim 1, **characterized in that**:
the at least two electrodes (171, 172, 271, 272, 371, 372, 373, 471, 472) are located on an outer surface of the cannula wall (132) or in the cannula wall (132).

3. The unilateral-driven medical device (100) with infusion and detection integrated of claim 2, **characterized in that**:
the at least two electrodes (171, 172, 271, 272, 371, 372, 373, 471, 472) are located on the outer surface of the cannula wall (132), and when the infusion cannula (130) is installed to the working position, the different electrodes (171, 172, 271, 272, 371, 372, 373, 471, 472) are directly electrically connected to the different electrically connective regions (121a, 121b, 121c), respectively.

4. The unilateral-driven medical device (100) with infusion and detection integrated of claim 3, **characterized in that**:
the at least two electrodes (271, 272) are located on a subcutaneous part of the outer surface of the cannula wall (132), and the outer surface of the cannula wall (132) is further provided with electrode leads (2710, 2720) electrically connected to the at least two electrodes (271, 272), and when the infusion cannula (130) is installed to the working position, the different electrode leads (2710, 2720) are electrically connected to the different electrically connective regions (121a, 121b), respectively.

5. The unilateral-driven medical device (100) with infusion and detection integrated of claim 2, **characterized in that**:
the infusion cannula (130) includes an inner layer cannula (170) and at least one outer layer cannula (180), and the at least one outer layer cannula (180) is disposed outside the inner layer cannula (170), and the inner layer cannula (170) is used for drug infusion.

6. The unilateral-driven medical device (100) with infusion and detection integrated of claim 5, **characterized in that**:
at least one of the at least two electrodes (471, 472) is provided between an outer wall of the inner layer cannula (170) and an outermost cannula,
when the infusion cannula (130) is installed to the working position, an electrode (472) located on the outer wall surface of the inner layer cannula (170) is entirely exposed in a subcutaneous tissue fluid, or covered in whole or in part by the at least one outer layer cannulas.

7. The unilateral-driven medical device (100) with infusion and detection integrated of claim 1, **characterized in that**:
the at least two electrodes (171, 172, 371, 372, 373) include a working electrode (171, 371) and an auxiliary electrode (172, 372, 373), and the number of the working electrode and the auxiliary electrode is one or more than one, respectively.

8. The unilateral-driven medical device (100) with infusion and detection integrated of claim 7, **characterized in that**:
a plurality of electrodes (371, 372, 373) form one or more electrode combination, each electrode combination comprising the working electrode (371) and the auxiliary electrode (372, 373), the program unit (120) choosing the one or more electrode combination to detect the analyte data in the body fluid.

9. The unilateral-driven medical device (100) with infusion and detection integrated of claim 1, **characterized in that**:
the input end (121) is an elastic member, and the elastic member comprises one of or a combination of a conductive strip, an oriented conductive silica gel, a conductive ring and a conductive ball.

10. The unilateral-driven medical device (100) with infusion and detection integrated of claim 1, **characterized in that**:
the drug infusion unit (110) includes a plurality of infusion subunits (110a, 110b), the plurality of infusion subunits (110a, 110b) being electrically connected to the output ends (122a, 122b), respectively, and the program unit (120) controlling whether each infusion subunit (110a, 110b) delivers the drug.

11. The unilateral-driven medical device (100) with infusion and detection integrated of claim 1, **characterized in that**:
the unilateral-driven medical device (100) with infusion and detection integrated is composed of a plurality of parts, the drug infusion unit and (110) the program unit (120) are arranged in different parts, and the different parts are connected by waterproof plugs.

12. The unilateral-driven medical device (100) with infusion and detection integrated of claim 1, **characterized in that**:
the at least one driving unit (1250a, 1250b, 1350a, 1350b, 1450, 1550) includes two driving portions (1251a, 1251b, 1351a, 1351b, 1451a, 1451b, 1551a, 1551b), and under a cooperative operation of the power unit (1180) and the reset unit, (1170) the two driving portions (1251a, 1251b, 1351a, 1351b, 1451a, 1451b, 1551a, 1551b) are capable of alternately pushing the wheel teeth (1241, 1341, 1441a, 1441b, 1541).

13. The unilateral-driven medical device (100) with infusion and detection integrated of claim 12, **characterized in that**:
the two driving portions (1251a, 1251b, 1351a, 1351b, 1551a, 1551b) alternately push the wheel teeth (1241, 1341, 1541) disposed on the same driving wheel (1240, 1340, 1540).

14. The unilateral-driven medical device (100) with infusion and detection integrated of claim 12, **characterized in that**:
the driving wheel includes two sub-wheels (1440a, 1440b) provided with the wheel teeth (1441a, 1441b), and the two driving portions (1451a, 1451b) respectively alternately push the wheel teeth (1441a, 1441b) disposed on different sub-wheels (1440a, 1440b).

## Patentansprüche

1. Einseitig angetriebenes medizinisches Gerät (100) mit integrierter Infusion und Detektion, **dadurch gekennzeichnet, dass** es umfasst:
eine Arzneimittelinfusionseinheit (110), umfassend:
mindestens eine Arzneimittelspeichereinheit (1110);
eine Schraube (1130), die mit einem Kolben (1120) bzw. einem Antriebsrad (1140, 1240, 1340, 1540) verbunden ist, das mit Radzähnen (1141, 1241, 1341, 1441a, 1441b, 1541) versehen ist, wobei das Antriebsrad (1140, 1240, 1340, 1540) die Schraube (1130) durch Drehung antreibt, um sich zu bewegen und den Kolben (1120), der in der mindestens einen Arzneimittelspeichereinheit (1110) vorgesehen ist, nach vorne zu drücken;
mindestens eine Antriebseinheit (1150, 1250a, 1250b, 1350a, 1350b, 1450, 1550), die mit dem Antriebsrad (1140, 1240, 1340, 1540) zusammenwirkt, wobei die mindestens eine Antriebseinheit (1150, 1250a, 1250b, 1350a, 1350b, 1450, 1550) mindestens einen Antriebsabschnitt (1151, 1251a, 1251b, 1351a, 1351b, 1451a, 1451b, 1551a, 1551b) umfasst;
eine Betätigungseinheit (1180) und eine Rücksetzeinheit (1170), die mit der mindestens einen Antriebseinheit (1150, 1250a, 1250b, 1350a, 1350b, 1450, 1550) verbunden sind, wobei:
wenn die Betätigungseinheit (1180) eine Kraft auf die mindestens eine Antriebseinheit (1150, 1250a, 1250b, 1350a, 1350b, 1450, 1550) ausübt, sich die mindestens eine Antriebseinheit (1150, 1250a, 1250b, 1350a, 1350b, 1450, 1550) um eine Schwenkachse (1160) dreht und den mindestens einen Antriebsabschnitt (1151, 1251a, 1251b, 1351a, 1351b, 1451a, 1451b, 1551a, 1551b) antreibt, um die Radzähne (1141, 1241, 1341, 1441a, 1441b, 1541) zu drücken, wodurch das Antriebsrad (1140, 1240, 1340, 1540) gedreht wird;
wenn die Rücksetzeinheit (1170) eine Kraft allein auf die mindestens eine Antriebseinheit (1150, 1250a, 1250b, 1350a, 1350b, 1450, 1550) ausübt, führt die mindestens eine Antriebseinheit (1150, 1250a, 1250b, 1350a, 1350b, 1450, 1550) eine Rücksetzdrehung durch, ohne dass der mindestens eine Antriebsabschnitt (1151, 1251a, 1251b, 1351a, 1351b, 1451a, 1451b, 1551a, 1551b) die Radzähne (1141, 1241, 1341, 1441a, 1441b, 1541) drückt, wodurch das Antriebsrad (1140, 1240, 1340, 1540) aufhört, sich zu drehen;
eine Programmeinheit (120), umfassend ein Eingangsende (121) und ein Ausgangsende (122), wobei das Eingangsende (121) eine Vielzahl von elektrisch verbindbaren Bereichen (121a, 121b, 121c) zum Empfangen von Signalen von Analyt-Daten in einer Körperflüssigkeit umfasst, wobei, nachdem das Ausgangsende (122) elektrisch mit der Betätigungseinheit (1180) oder der Rücksetzeinheit (1170) verbunden ist, die Programmeinheit (120) gemäß empfangenen Signalen der Analyt-Daten in der Körperflüssigkeit über die Betätigungseinheit (1180) oder die Rücksetzeinheit (1170) die Drehung der mindestens einen Antriebseinheit (1150, 1250a, 1250b, 1350a, 1350b, 1450, 1550) steuert, um eine Arzneimittelinfusion zu bewirken; und
eine Infusionskanüle (130), die mit mindestens zwei Elektroden (171, 172, 271, 272, 371, 372, 373, 471, 472) versehen ist, wobei die Infusionskanüle (130) einen Arzneimittelinfusionskanal bildet, wobei die mindestens zwei Elektroden (171, 172, 271, 272, 371, 372, 373, 471, 472) an / in einer Kanülenwand (132) angeordnet sind, wenn die Infusionskanüle (130) in einer Arbeitsposition angeordnet ist, die Infusionskanüle (130) mit der Arzneimittelinfusionseinheit (110) verbunden ist, ein Arzneimittel dann durch die Infusionskanüle (130) in einen Körper injiziert werden kann, und die verschiedenen Elektroden (171, 172, 271, 272, 371, 372, 373, 471, 472) jeweils mit den verschiedenen elektrisch leitenden Bereichen (121a, 121b, 121c) elektrisch verbunden sind, wodurch Signale der Analyt-Daten in der Körperflüssigkeit in die Programmeinheit (120) eingegeben werden,
wobei die Elektrode (472), die sich an der Außenwandfläche der inneren Kanülenschicht (170) befindet, ganz oder teilweise von der mindestens einen äußeren Kanülenschicht (180) abgedeckt ist, wobei das Material der Wände der mindestens einen äußeren Kanülenschicht (180) eine durchlässige Membran oder eine halbdurchlässige Membran ist.

2. Einseitig angetriebenes medizinisches Gerät (100) mit integrierter Infusion und Detektion nach Anspruch 1, **dadurch gekennzeichnet, dass**:
sich die mindestens zwei Elektroden (171, 172, 271, 272, 371, 372, 373, 471, 472) an einer Außenfläche der Kanülenwand (132) oder in der Kanülenwand (132) befinden.

3. Einseitig angetriebenes medizinisches Gerät (100) mit integrierter Infusion und Detektion nach Anspruch 2, **dadurch gekennzeichnet, dass**:
sich die mindestens zwei Elektroden (171, 172, 271, 272, 371, 372, 373, 471, 472) an der Außenfläche der Kanülenwand (132) befinden, und wenn die Infusionskanüle (130) in der Arbeitsposition angeordnet ist, die verschiedenen Elektroden (171, 172, 271, 272, 371, 372, 373, 471, 472) direkt elektrisch mit den verschiedenen elektrisch leitenden Bereichen (121a, 121b, 121c) verbunden sind.

4. Einseitig angetriebenes medizinisches Gerät (100) mit integrierter Infusion und Detektion nach Anspruch 3, **dadurch gekennzeichnet, dass**:
sich die mindestens zwei Elektroden (271, 272) auf einem subkutanen Teil der Außenfläche der Kanülenwand (132) befinden und die Außenfläche der Kanülenwand (132) ferner mit Elektrodenleitungen (2710, 2720) versehen ist, die elektrisch mit den mindestens zwei Elektroden (271, 272) verbunden sind, und wenn die Infusionskanüle (130) in der Arbeitsposition angeordnet ist, die verschiedenen Elektrodenleitungen (2710, 2720) elektrisch mit den verschiedenen elektrisch leitenden Bereichen (121a, 121b) verbunden sind.

5. Einseitig angetriebenes medizinisches Gerät (100) mit integrierter Infusion und Detektion nach Anspruch 2, **dadurch gekennzeichnet, dass**:
die Infusionskanüle (130) eine innere Kanülenschicht (170) und mindestens eine äußere Kanülenschicht (180) umfasst, und die mindestens eine äußere Kanülenschicht (180) außerhalb der inneren Kanülenschicht (170) angeordnet ist, und die innere Kanülenschicht (170) zur Arzneimittelinfusion verwendet wird.

6. Einseitig angetriebenes medizinisches Gerät (100) mit integrierter Infusion und Detektion nach Anspruch 5, **dadurch gekennzeichnet, dass**:
mindestens eine der mindestens zwei Elektroden (471, 472) zwischen einer Außenwand der inneren Kanülenschicht (170) und einer äußersten Kanüle vorgesehen ist,
wenn die Infusionskanüle (130) in der Arbeitsposition angeordnet ist, eine Elektrode (472), die sich auf der Außenwandfläche der inneren Kanülenschicht (170) befindet, vollständig in einer subkutanen Gewebeflüssigkeit freiliegt oder ganz oder teilweise von der mindestens einen äußeren Kanülenschicht (180) abgedeckt ist.

7. Einseitig angetriebenes medizinisches Gerät (100) mit integrierter Infusion und Detektion nach Anspruch 1, **dadurch gekennzeichnet, dass**:
die mindestens zwei Elektroden (171, 172, 371, 372, 373) eine Arbeitselektrode (171, 371) und eine Hilfselektrode (172, 372, 373) umfassen und die Anzahl der Arbeitselektroden bzw. der Hilfselektroden eine oder mehr als eine beträgt.

8. Einseitig angetriebenes medizinisches Gerät (100) mit integrierter Infusion und Detektion nach Anspruch 7, **dadurch gekennzeichnet, dass**:
eine Vielzahl von Elektroden (371, 372, 373) eine oder mehrere Elektrodenkombinationen bilden, wobei jede Elektrodenkombination die Arbeitselektrode (371) und die Hilfselektrode (372, 373) umfasst, wobei die Programmeinheit (120) eine oder mehrere Elektrodenkombinationen auswählt, um die Analyt-Daten in der Körperflüssigkeit zu erfassen.

9. Einseitig angetriebenes medizinisches Gerät (100) mit integrierter Infusion und Detektion nach Anspruch 1, **dadurch gekennzeichnet, dass**:
das Eingangsende (121) ein elastisches Element ist und das elastische Element eines oder eine Kombination aus einem leitfähigen Streifen, einem orientierten leitfähigen Silikagel, einem leitfähigen Ring und einer leitfähigen Kugel umfasst.

10. Einseitig angetriebenes medizinisches Gerät (100) mit integrierter Infusion und Detektion nach Anspruch 1, **dadurch gekennzeichnet, dass**:
die Arzneimittelinfusionseinheit (110) eine Vielzahl von Infusionsuntereinheiten (110a, 110b) umfasst, wobei die Vielzahl von Infusionsuntereinheiten (110a, 110b) jeweils elektrisch mit den Ausgangsenden (122a, 122b) verbunden ist und die Programmeinheit (120) steuert, ob jede Infusionsuntereinheit (110a, 110b) das Arzneimittel abgibt.

11. Einseitig angetriebenes medizinisches Gerät (100) mit integrierter Infusion und Detektion nach Anspruch 1, **dadurch gekennzeichnet, dass**:
das einseitig angetriebene medizinische Gerät (100) mit integrierter Infusion und Detektion aus einer Vielzahl von Teilen gebildet ist, die Arzneimittelinfusionseinheit (110) und die Programmeinheit (120) in verschiedenen Teilen angeordnet sind und die verschiedenen Teile durch wasserdichte Stecker verbunden sind.

12. Einseitig angetriebenes medizinisches Gerät (100) mit integrierter Infusion und Detektion nach Anspruch 1, **dadurch gekennzeichnet, dass**:
die mindestens eine Antriebseinheit (1250a, 1250b, 1350a, 1350b, 1450, 1550) zwei Antriebsabschnitte (1251a, 1251b, 1351a, 1351b, 1451a, 1451b, 1551a, 1551b) umfasst und unter einem zusammenwirkenden Betrieb der Betätigungseinheit (1180) und der Rücksetzeinheit (1170) die beiden Antriebsabschnitte (1251a, 1251b, 1351a, 1351b, 1451a, 1451b, 1551a, 1551b) in der Lage sind, die Radzähne (1241, 1341, 1441a, 1441b, 1541) abwechselnd zu drücken.

13. Einseitig angetriebenes medizinisches Gerät (100) mit integrierter Infusion und Detektion nach Anspruch 12, **dadurch gekennzeichnet, dass**:
die beiden Antriebsabschnitte (1251a, 1251b, 1351a, 1351b, 1551a, 1551b) die auf demselben Antriebsrad (1240, 1340, 1540) angeordneten Radzähne (1241, 1341, 1541) abwechselnd drücken.

14. Einseitig angetriebenes medizinisches Gerät (100) mit integrierter Infusion und Detektion nach Anspruch 12, **dadurch gekennzeichnet, dass**:
das Antriebsrad zwei Unterräder (1440a, 1440b) umfasst, die mit den Radzähnen (1441a, 1441b) versehen sind, und die beiden Antriebsabschnitte (1451a, 1451b) jeweils abwechselnd die auf verschiedenen Unterrädern (1440a, 1440b) angeordneten Radzähne (1441a, 1441b) drücken.

## Revendications

1. Dispositif médical à entraînement unilatéral (100) avec perfusion et détection intégrées, **caractérisé en ce qu'**il comprend :
une unité de perfusion de médicament (110), comprenant :
au moins une unité de stockage de médicament (1110) ;
une vis (1130) reliée à un piston (1120) et une roue d'entraînement (1140, 1240, 1340, 1540) pourvue de dents de roue (1141, 1241, 1341, 1441a, 1441b, 1541), respectivement, la roue d'entraînement (1140, 1240, 1340, 1540) entraînant la vis (1130) à se déplacer par rotation, poussant le piston (1120), prévu dans la au moins une unité de stockage de médicament (1110), vers l'avant ;
au moins une unité d'entraînement (1150, 1250a, 1250b, 1350a, 1350b, 1450, 1550) coopérant avec la roue d'entraînement (1140, 1240, 1340, 1540), au moins une unité d'entraînement (1150, 1250a, 1250b, 1350a, 1350b, 1450, 1550) comprend au moins une partie d'entraînement (1151, 1251a, 1251b, 1351a, 1351b, 1451a, 1451b, 1551a, 1551b) ; une unité d'alimentation (1180) et une unité de réinitialisation (1170) connectées au moins une unité d'entraînement (1150, 1250a, 1250b, 1350a, 1350b, 1450, 1550), dans laquelle :
lorsque l'unité d'alimentation (1180) exerce une force sur au moins une unité d'entraînement (1150, 1250a, 1250b, 1350a, 1350b, 1450, 1550), au moins une unité d'entraînement (1150, 1250a, 1250b, 1350a, 1350b, 1450, 1550) tourne autour d'un axe de pivotement (1160), entraînant au moins une partie d'entraînement (1151, 1251a, 1251b, 1351a, 1351b, 1451a, 1451b, 1551a, 1551b) pour pousser les dents de roue (1141, 1241, 1341, 1441a, 1441b, 1541), faisant ainsi tourner la roue d'entraînement (1140, 1240, 1340, 1540) ;
lorsque l'unité de réinitialisation (1170) exerce une force sur au moins une unité d'entraînement (1150, 1250a, 1250b, 1350a, 1350b, 1450, 1550) seule, au moins une unité d'entraînement (1150, 1250a, 1250b, 1350a, 1350b, 1450, 1550) effectue une rotation de réinitialisation sans qu'au moins une partie d'entraînement (1151, 1251a, 1251b, 1351a, 1351b, 1451a, 1451b, 1551a, 1551b) poussant les dents de roue (1141, 1241, 1341, 1441a, 1441b, 1541), ce qui fait que la roue d'entraînement (1140, 1240, 1340, 1540) cesse de tourner ;
une unité de programmation (120) comprenant une extrémité d'entrée (121) et une extrémité de sortie (122), l'extrémité d'entrée (121) comprenant une pluralité de zones de connexion électrique (121a, 121b, 121c) destinées à recevoir des signaux de données d'analyte dans un fluide corporel, après que l'extrémité de sortie (122) a été connectée électriquement à l'unité d'alimentation (1180) ou à l'unité de réinitialisation (1170), en fonction des signaux reçus des données d'analyte dans le fluide corporel, l'unité de programmation (120), par l'intermédiaire de l'unité d'alimentation (1180) ou de l'unité de réinitialisation (1170), commande la rotation de au moins une unité d'entraînement (1150, 1250a, 1250b, 1350a, 1350b, 1450, 1550) pour mettre en œuvre la perfusion de médicament ; et
une canule de perfusion (130) pourvue d'au moins deux électrodes (171, 172, 271, 272, 371, 372, 373, 471, 472), la canule de perfusion (130) est un canal de perfusion de médicament, les au moins deux électrodes (171, 172, 271, 272, 371, 372, 373, 471, 472) sont disposées sur/dans une paroi de canule (132), lorsque la canule de perfusion (130) est installée dans une position de travail, la canule de perfusion (130) est reliée à l'unité de perfusion de médicament (110), un médicament peut alors être injecté dans un corps à travers la canule de perfusion (130), et les différentes électrodes (171, 172, 271, 272, 371, 372, 373, 471, 472) sont connectées électriquement aux différentes zones de connexion électrique (121a, 121b, 121c) respectivement, entrant le signal des données d'analyte dans le fluide corporel à l'unité de programmation (120),
dans lequel l'électrode (472) située sur la surface de la paroi extérieure de la canule à couche intérieure (170) est recouverte en tout ou en partie par au moins une canule à couche externe (180), le matériau des parois de au moins une canule à couche externe (180) est une membrane perméable ou une membrane semi-perméable.

2. Dispositif médical à entraînement unilatéral (100) avec perfusion et détection intégrées selon la revendication 1, **caractérisé en ce que** :
les au moins deux électrodes (171, 172, 271, 272, 371, 372, 373, 471, 472) sont situées sur une surface extérieure de la paroi de la canule (132) ou dans la paroi de la canule (132).

3. Dispositif médical à entraînement unilatéral (100) avec perfusion et détection intégrées selon la revendication 2, **caractérisé en ce que** :
les au moins deux électrodes (171, 172, 271, 272, 371, 372, 373, 471, 472) sont situées sur la surface extérieure de la paroi de la canule (132), et lorsque la canule de perfusion (130) est installée en position de travail, les différentes électrodes (171, 172, 271, 272, 371, 372, 373, 471, 472) sont directement connectées électriquement aux différentes zones de connexion électrique (121a, 121b, 121c), respectivement.

4. Dispositif médical à entraînement unilatéral (100) avec perfusion et détection intégrées selon la revendication 3, **caractérisé en ce que** :
les au moins deux électrodes (271, 272) sont situées sur une partie sous-cutanée de la surface extérieure de la paroi de la canule (132), et la surface extérieure de la paroi de la canule (132) est en outre pourvue de fils d'électrodes (2710, 2720) connectés électriquement aux au moins deux électrodes (271, 272), et lorsque la canule de perfusion (130) est installée en position de travail, les différents fils d'électrode (2710, 2720) sont connectés électriquement aux différentes zones de connexion électrique (121a, 121b), respectivement.

5. Dispositif médical à entraînement unilatéral (100) avec perfusion et détection intégrées selon la revendication 2, **caractérisé en ce que** :
la canule de perfusion (130) comprend une canule à couche interne (170) et au moins une canule à couche externe (180), et au moins une canule à couche externe (180) est disposée à l'extérieur de la canule à couche interne (170), et la canule à couche interne (170) est utilisée pour la perfusion de médicaments.

6. Dispositif médical à entraînement unilatéral (100) avec perfusion et détection intégrées selon la revendication 5, **caractérisé en ce que** :
au moins une des au moins deux électrodes (471, 472) est prévue entre une paroi extérieure de la canule à couche interne (170) et une canule la plus externe,
lorsque la canule de perfusion (130) est installée en position de travail, une électrode (472) située sur la surface de la paroi extérieure de la canule à couche interne (170) est entièrement exposée dans un fluide tissulaire sous-cutané, ou recouverte en tout ou en partie par au moins une canule de la couche externe.

7. Dispositif médical à entraînement unilatéral (100) avec perfusion et détection intégrées selon la revendication 1, **caractérisé en ce que** :
les au moins deux électrodes (171, 172, 371, 372, 373) comprennent une électrode de travail (171, 371) et une électrode auxiliaire (172, 372, 373), et le nombre d'électrodes de travail et d'électrodes auxiliaires est respectivement égal à un ou supérieur à un.

8. Dispositif médical à entraînement unilatéral (100) avec perfusion et détection intégrées selon la revendication 7, **caractérisé en ce que** :
une pluralité d'électrodes (371, 372, 373) forme une ou plusieurs combinaisons d'électrodes, chaque combinaison d'électrodes comprenant l'électrode de travail (371) et l'électrode auxiliaire (372, 373), l'unité de programmation (120) choisissant la ou les combinaisons d'électrodes pour détecter les données d'analyte dans le fluide corporel.

9. Dispositif médical à entraînement unilatéral (100) avec perfusion et détection intégrées selon la revendication 1, **caractérisé en ce que** :
l'extrémité d'entrée (121) est un élément élastique, et l'élément élastique comprend l'un ou une combinaison d'une bande conductrice, d'un gel de silice conducteur orienté, d'un anneau conducteur et d'une bille conductrice.

10. Dispositif médical à entraînement unilatéral (100) avec perfusion et détection intégrées selon la revendication 1, **caractérisé en ce que** :
l'unité de perfusion de médicament (110) comprend une pluralité de sous-unités de perfusion (110a, 110b), la pluralité de sous-unités de perfusion (110a, 110b) étant connectées électriquement aux extrémités de sortie (122a, 122b), respectivement, et l'unité de programmation (120) contrôle si chaque sous-unité de perfusion (110a, 110b) administre le médicament.

11. Dispositif médical à entraînement unilatéral (100) avec perfusion et détection intégrées selon la revendication 1, **caractérisé en ce que** :
le dispositif médical à entraînement unilatéral (100) avec perfusion et détection intégrées est composé d'une pluralité de parties, l'unité de perfusion de médicament (110) et l'unité de programmation (120) sont disposées dans des parties différentes, et les différentes parties sont connectées par des fiches étanches.

12. Dispositif médical à entraînement unilatéral (100) avec perfusion et détection intégrées de la revendication 1, **caractérisé en ce que** :
la au moins une unité d'entraînement (1250a, 1250b, 1350a, 1350b, 1450, 1550) comprend deux parties d'entraînement (1251a, 1251b, 1351a, 1351b, 1451a, 1451b, 1551a, 1551b), et sous l'action conjointe de l'unité d'alimentation (1180) et de l'unité de réinitialisation (1170), les deux parties d'entraînement (1251a, 1251b, 1351a, 1351b, 1451a, 1451b, 1551a, 1551b) sont capables de pousser alternativement les dents de roue (1241, 1341, 1441a, 1441b, 1541).

13. Dispositif médical à entraînement unilatéral (100) avec perfusion et détection intégrées selon la revendication 12, **caractérisé en ce que** :
les deux parties d'entraînement (1251a, 1251b, 1351a, 1351b, 1551a, 1551b) poussent alternativement les dents de roue (1241, 1341, 1541) disposées sur la même roue d'entraînement (1240, 1340, 1540).

14. Dispositif médical à entraînement unilatéral (100) avec perfusion et détection intégrées selon la revendication 12, **caractérisé en ce que** :
la roue d'entraînement comprend deux sous-roues (1440a, 1440b) pourvues des dents de roue (1441a, 1441b), et les deux parties d'entraînement (1451a, 1451b) poussent respectivement en alternance les dents de roue (1441a, 1441b) disposées sur différentes sous-roues (1440a, 1440b).
